(19) Europäisches Patentamt
European Patent Office
Office européen des brevets

(11) **EP 1 604 635 A2**

(12) **DEMANDE DE BREVET EUROPEEN**

(43) Date de publication:
**14.12.2005 Bulletin 2005/50**

(51) Int Cl.[7]: **A61K 7/025**, A61K 7/48, A61K 7/02

(21) Numéro de dépôt: **05291039.5**

(22) Date de dépôt: **13.05.2005**

(84) Etats contractants désignés:
**AT BE BG CH CY CZ DE DK EE ES FI FR GB GR HU IE IS IT LI LT LU MC NL PL PT RO SE SI SK TR**
Etats d'extension désignés:
**AL BA HR LV MK YU**

(30) Priorité: **08.06.2004 FR 0406172**

(71) Demandeur: **L'OREAL**
**75008 Paris (FR)**

(72) Inventeurs:
- **Lebre, Caroline**
  **94320 Thiais (FR)**
- **Lion, Bertrand**
  **75012 Paris (FR)**

(74) Mandataire: **Chantraine, Sylvie Hélène**
**L'OREAL - D.I.P.I.**
**25-29, Quai Aulagnier**
**92600 Asnieres (FR)**

(54) **Composition comprenant des particules de polymère dispersées dans une phase grasse et une huile apolaire**

(57) La présente invention a pour objet une composition cosmétique contenant des particules d'au moins un polymère dispersées dans une phase grasse,

- ladite phase grasse étant exempte d'huile volatile ou contenant moins de 50% en poids d'huile volatile par rapport au poids de ladite phase grasse, et
- ladite phase grasse liquide contenant au moins 5% en poids d'au moins une huile peu polaire ou apolaire.

L'huile apolaire ou peu polaire est de préférence une huile apolaire hydrocarbonée non volatile.



Printed by Jouve, 75001 PARIS (FR)

## Description

La présente invention se rapporte à une composition cosmétique, notamment pour le maquillage ou le soin de la peau, y compris du cuir chevelu, aussi bien du visage que du corps humain, des lèvres ou des phanères des êtres humains, comme les cheveux, les cils, ou les sourcils, comprenant des particules de polymère dispersées dans une phase grasse.

Il a été décrit dans la demande EP 1 002 528 des compositions non transfert contenant une dispersion de particules de polymère dans une huile volatile. Ces compositions contiennent majoritairement une huile volatile, et également des huiles siliconées non volatiles et des huiles hydrocarbonées non volatiles. Les huiles hydrocarbonées non volatiles sont en très faible quantité, de l'ordre de 3%.

Il subsiste un besoin d'un produit cosmétique brillant et non migrant contenant une dispersion de particules de polymère en phase grasse. De façon surprenante, les inventeurs ont trouvé que l'utilisation d'une telle dispersion de particules de polymère en association avec une quantité minimale d'huile apolaire ou peu polaire, en présence d'une quantité limitée d'huile(s) volatile(s) permet d'obtenir une composition brillante non migrante.

En outre, les compositions brillantes connues ont souvent l'inconvénient d'être de mauvaise tenue. Or, les femmes sont aujourd'hui à la recherche de produits, notamment de maquillage des lèvres ou des paupières, brillants qui ne perdent pas en tenue. La composition brillante selon l'invention répond à ce besoin puisqu'elle présente également une bonne tenue.

La composition de l'invention peut en particulier constituer un produit capillaire ou un produit de maquillage du corps, des lèvres ou des phanères d'êtres humains ayant des propriétés de soin et/ou de traitement. Elle constitue notamment un rouge à lèvres ou un brillant à lèvres, un fard à paupières, un produit pour tatouage, un mascara, un eye-liner, un produit de bronzage artificiel de la peau, une crème de soin ou de protection éventuellement teintée, un produit de coloration ou de soin des cheveux.

Selon un mode de réalisation, la composition selon l'invention présente l'avantage d'avoir une bonne tenue, d'être non collante, de ne pas migrer en dehors du dépôt initial. Lorsque la composition est appliquée sur la peau ou les lèvres, elle procure également une sensation de confort à l'application et une fois déposée.

De façon plus précise, l'invention a pour objet une composition cosmétique contenant des particules d'au moins un polymère dispersées dans une phase grasse,

- ladite phase grasse étant exempte d'huile volatile ou contenant moins de 50% en poids d'huile volatile par rapport au poids de ladite phase grasse, et
- ladite phase grasse liquide contenant au moins 5% en poids d'au moins une huile peu polaire ou apolaire.

  La phase grasse contient avantageusement au moins 10% en poids d'au moins une huile apolaire ou peu polaire.

  Avantageusement, le polymère est tel, que lorsqu'il est dispersé en quantité suffisante dans la phase grasse, la brillance moyenne à 20° d'un dépôt de ladite composition, une fois étalée sur un support, est supérieure ou égale à 30 sur 100.

  Par « brillance moyenne », on désigne la brillance telle qu'elle peut être mesurée à l'aide d'un brillancemètre, de manière conventionnelle par la méthode suivante.

  Sur une carte de contraste de marque LENETA et de référence FORM 1A PENOPAC, on étale une couche comprise entre 50 µm et 150 µm d'épaisseur de la composition à l'aide d'un étaleur automatique. La couche recouvre au moins le fond blanc de la carte. Lorsque la composition est solide, on la fait fondre si nécessaire sur la carte après l'avoir étalée afin qu'elle recouvre le fond blanc.

  On laisse sécher le dépôt 24 heures à une température de 30°C, puis on procède à la mesure de la brillance à 20° sur le fond blanc à l'aide d'un brillancemètre de marque BYK GARDNER et de référence microTRI-GLOSS. Cette mesure (comprise entre 0 et 100) est répétée au moins trois fois, et la brillance moyenne est la moyenne des au moins trois mesures effectuées.

  La brillance moyenne de la composition mesurée à 20° est avantageusement supérieure

ou égale à 30, mieux encore supérieure ou égale à 35, mieux encore supérieure ou égale à 40, mieux encore supérieure ou égale à 45, mieux encore supérieure ou égale à 50 sur 100, mieux encore, supérieure ou égale à 55, mieux encore, supérieure ou égale à 60.

De préférence, la brillance moyenne de la composition, une fois étalée sur un support, mesurée à 60° est supérieure ou égale à 50, mieux encore, supérieure ou égale à 60, mieux encore, supérieure ou égale à 65, mieux encore, supérieure ou égale à 70, mieux encore, supérieure ou égale à 75, mieux encore, supérieure ou égale à 80, mieux encore, supérieure ou égale à 85 ou mieux encore, supérieure ou égale à 90 sur 100.

On procède à la mesure de la brillance moyenne à 60° comme décrit précédemment en effectuant la mesure à 60° et non pas 20°.

Selon un mode de mise en oeuvre, la brillance moyenne de la composition mesurée à 20° est de préférence supérieure ou égale à 35, de préférence 40, 45 ou 50 sur 100, et/ou la brillance moyenne de la composition mesurée à 60° est de préférence supérieure ou égale à 65, 70 ou 75 sur 100. Dans ce mode de mise en oeuvre, la composition constitue avantageusement un rouge à lèvres liquide.
L'invention se rapporte également à l'utilisation cosmétique de la composition définie ci-dessus pour améliorer la brillance d'un dépôt sur la peau et/ou les lèvres et/ou les phanères, en particulier un maquillage.
Par "phase grasse", au sens de la demande, on entend tout milieux non aqueux.
De préférence, la phase grasse est au moins en partie liquide à température ambiante (25°C) et pression atmosphérique (760 mm de Hg), et est composée d'un ou plusieurs corps gras liquides à température ambiante, appelés aussi huiles.
L'invention se rapporte également à l'utilisation cosmétique de la composition définie ci-dessus pour conférer de la brillance et de la non migration à un dépôt sur la peau et/ou les lèvres et/ou les phanères, en particulier un dépôt de maquillage.

**Polymère en dispersion**

Selon l'invention le polymère est un solide insoluble dans la phase grasse de la composition à une température ambiante, par exemple, d'environ 25°C. Le polymère est également insoluble dans la phase grasse à sa température de ramollissement, à l'inverse d'une cire qui est soluble dans la phase grasse à une température seupérieure à sa température de fusion. En ce sens, le polymère n'est pas une cire.
Le polymère en dispersion permet, en outre, la formation d'un dépôt notamment continu et homogène et/ou est caractérisé par l'enchevêtrement des chaînes polymériques.
La composition selon l'invention comprend avantageusement au moins une dispersion stable de particules de polymère essentiellement sphériques d'un ou plusieurs polymères, dans la phase grasse.
Ces dispersions peuvent notamment se présenter sous forme de nanoparticules de polymères en dispersion stable dans ladite phase organique liquide. Les nanoparticules sont de préférence d'une taille moyenne comprise entre 5 et 800 nm, et mieux entre 50 et 500 nm. Il est toutefois possible d'obtenir des tailles de particules de polymère allant jusqu'à 1μm.
De préférence, les particules de polymères en dispersion sont insolubles dans les alcools hydrosolubles tels que, par exemple, l'éthanol.
Les polymères en dispersion utilisables dans la composition de l'invention ont de préférence un poids moléculaire de l'ordre de 2000 à 10 000 000 g/mol, et une Tg de -100°C à 300°C et mieux de -50° à 100°C, de préférence de -10°C à 50°C.
De préférence le polymère est filmifiable. Il est possible d'utiliser des polymères filmifiables, de préférence ayant une Tg basse, inférieure ou égale à la température de la peau et notamment inférieure ou égale à 40°C.
Par polymère "filmifiable", on entend un polymère apte à former à lui seul ou en présence d'un agent auxiliaire de filmification, un film continu et adhérent sur un support, notamment sur les matières kératiniques, et de préférence un film cohésif et mieux encore, un film dont la cohésion et les propriétés mécaniques sont telles que ledit film peut être isolé dudit support.
Il est toutefois possible d'utiliser un polymère non filmifiable. Par « polymère non filmifiable », on entend un polymère non capable de former seul, un film isolable.
Parmi les polymères filmifiables, on peut citer des homopolymères ou des copolymères radicalaires, acryliques ou vinyliques, de préférence ayant une Tg inférieure ou égale à 40°C et notamment allant de -10° à 30°C, utilisés seul ou en mélange.
Parmi les polymères non filmifiables, on peut citer des homopolymères ou copolymères radicalaires, vinyliques ou acryliques, éventuellement réticulés, ayant de préférence une Tg supérieure à 40°C et notamment allant de 45° à 150°C, utilisés seul ou en mélange.
Par polymère radicalaire, on entend un polymère obtenu par polymérisation de monomères à insaturation notamment éthylénique, chaque monomère étant susceptible de s'homopolymériser (à l'inverse des polycondensats). Les polymères radicalaires peuvent être notamment des polymères, ou des copolymères, vinyliques, notamment des polymères acryliques.
Les polymères acryliques peuvent résulter de la polymérisation de monomères à insaturation éthylénique ayant au moins un groupement acide et/ou des esters de ces monomères acides et/ou des amides de ces acides.
On préfère dans le cadre de la présente invention les copolymères méth(acrylique)s/méthacrylate(s), en particulier les copolymères acrylique/acrylate tels que le ratio massique des motifs acryliques et des motifs acrylates est compris entre 0,1 et 40%, de préférence entre 2 et 30%, de préférence encore entre 5 et 20%.
Comme monomère porteur de groupement acide, on peut utiliser des acides carboxyliques insaturés α,β-éthyléniques tels que l'acide acrylique, l'acide méthacrylique, l'acide crotonique, l'acide maléique, l'acide itaconique.

On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), comme les (méth)acrylates d'alkyle, en particulier d'alkyle en $C_1$-$C_{20}$, de préférence en $C_1$-$C_8$, les (méth)acrylates d'aryle, en particulier d'aryle en $C_6$-$C_{10}$, les (méth)acrylates d'hydroxyalkyle, en particulier d'hydroxyalkyle en $C_2$-$C_6$. Comme (méth)acrylates d'alkyle, on peut citer le (méth)acrylate de méthyle, d'éthyle, de butyle, d'isobutyle, d'éthyl-2 hexyle et de lauryle. Comme (méth)acrylates d'hydroxyalkyle, on peut citer le (méth)acrylate d'hydroxyéthyle, le (méth)acrylate de 2-hydroxypropyle. Comme (méth)acrylates d'aryle, on peut citer l'acrylate de benzyle ou de phényle.

Les esters de l'acide (méth)acrylique particulièrement préférés sont les (méth)acrylates d'alkyle.

Comme polymère radicalaire, on utilise de préférence les copolymères d'acide (méth)acrylique et de (méth) acrylate d'alkyle, notamment d'alkyle en $C_1$-$C_4$. Plus préférentiellement, on peut utiliser les acrylates de méthyle éventuellement copolymérisés avec l'acide acrylique.

Comme amides des monomères acides, on peut citer les (méth)acrylamides, et notamment les N-alkyl (méth) acrylamides, en particulier d'alkyle en $C_2$-$C_{12}$ tels que le N-éthyl acrylamide, le N-t-butyl acrylamide, le N-octyl acrylamide ; les N- dialkyl ($C_1$-$C_4$) (méth)acrylamides.

Les polymères acryliques peuvent également résulter de la polymérisation de monomères à insaturation éthylénique ayant au moins un groupe amine, sous forme libre ou bien partiellement ou totalement neutralisée, ou bien encore partiellement ou totalement quaternisée. De tels monomères peuvent être par exemple le (méth)acrylate de diméthylaminoéthyle, le méthacrylamide de diméthylaminoéthyle, la vinylamine, la vinylpyridine, le chlorure de diallyldiméthylammonium.

Les polymères vinyliques peuvent également résulter de l'homopolymérisation ou de la copolymérisation d'au moins un monomère choisi parmi les esters vinyliques et les monomères styrèniques. En particulier, ces monomères peuvent être polymérisés avec des monomères acides et/ou leurs esters et/ou leurs amides, tels que ceux mentionnés précédemment. Comme exemple d'esters vinyliques, on peut citer l'acétate de vinyle, le propionate de vinyle, le néodécanoate de vinyle, le pivalate de vinyle, le benzoate de vinyle et le t-butyl benzoate de vinyle. Comme monomères styréniques, on peut citer le styrène et l'alpha-méthyl styrène.

La liste des monomères donnée n'est pas limitative et il est possible d'utiliser tout monomère connu de l'homme du métier entrant dans les catégories de monomères acryliques et vinyliques (y compris les monomères modifiés par une chaîne siliconée).

Comme autres monomères vinyliques utilisables, on peut encore citer :

- la N-vinylpyrrolidone, la vinylcaprolactame, les vinyl N-alkyl($C_1$-$C_6$) pyrroles, les vinyl-oxazoles, les vinyl-thiazoles, les vinylpyrimidines, les vinylimidazoles,
- les oléfines tels que l'éthylène, le propylène, le butylène, l'isoprène, le butadiène.

Le polymère vinylique peut être réticulé à l'aide d'un ou plusieurs monomères difonctionnels, notamment comprenant au moins deux insaturations éthyléniques, tel que le diméthacrylate d'éthylène glycol ou le phtalate de diallyle.

De façon non limitative, les polymères en dispersion de l'invention peuvent être choisis parmi, les polymères ou copolymères suivants : polyuréthanes, polyuréthanes-acryliques, polyurées, polyurée-polyuréthanes, polyester-polyuréthanes, polyéther-polyuréthanes, polyesters, polyesters amides, alkydes ; polymères ou copolymères acryliques et/ou vinyliques ; copolymères acryliques-silicone ; polyacrylamides ; polymères siliconés comme les polyuréthanes ou acryliques siliconés, polymères fluorés et leurs mélanges.

Le ou les polymères en dispersion dans la phase grasse peuvent représenter en matière sèche de 5 à 40% du poids de la composition, de préférence de 5 à 35 % et mieux de 8 à 30%.

Selon un mode de mise en oeuvre, les particules de polymère en dispersion sont stabilisées en surface par un stabilisant solide à température ambiante. Dans ce cas, la quantité en matière sèche de la dispersion représente la quantité totale de polymère + stabilisant, sachant que la quantité de polymère ne peut être inférieure à 5%.

On choisit de préférence d'utiliser une dispersion de particules d'au moins un polymère filmifiable.

*Stabilisant*

Les particules de polymère en milieu organique sont avantageusement stabilisées en surface, au fur et à mesure de la polymérisation, grâce à un stabilisant qui peut être un polymère séquencé, un polymère greffé, et/ou un polymère statistique, seul ou en mélange. La stabilisation peut être effectuée par tout moyen connu, et en particulier par ajout direct du polymère séquencé, polymère greffé et/ou polymère statistique, lors de la polymérisation.

Le stabilisant est de préférence également présent dans le mélange avant polymérisation. Toutefois, il est également possible de l'ajouter en continu, notamment lorsqu'on ajoute également les monomères en continu.

On peut utiliser 2-30% en poids de stabilisant par rapport au mélange initial de monomères, et de préférence 5-20% en poids.

Lorsqu'on utilise un polymère greffé et/ou séquencé en tant que stabilisant, on choisit le solvant de synthèse de telle manière qu'au moins une partie des greffons ou séquences dudit polymère-stabilisant soit soluble dans ledit solvant, l'autre partie des greffons ou séquences n'y étant pas soluble. Le polymère-stabilisant utilisé lors de la polymérisation doit être soluble, ou dispersible, dans le solvant de synthèse. De plus, on choisit de préférence un stabilisant dont les séquences ou greffons insolubles présentent une certaine affinité pour le polymère formé lors de la polymérisation.

Parmi les polymères greffés, on peut citer les polymères siliconés greffés avec une chaîne hydrocarbonée ; les polymères hydrocarbonés greffés avec une chaîne siliconée.

Conviennent également les copolymères greffés ayant par exemple un squelette insoluble de type polyacrylique avec des greffons solubles de type acide poly-12-(hydroxystéarique).

Ainsi on peut utiliser des copolymères blocs greffés ou séquencés comprenant au moins un bloc de type polyorganosiloxane et au moins un bloc d'un polymère radicalaire, comme les copolymères greffés de type acrylique/ silicone qui peuvent être employés notamment lorsque le milieu le milieu de synthèse puis la phase organique de la première composition contient une phase siliconée.

On peut aussi utiliser des copolymères blocs greffés ou séquencés comprenant au moins un bloc de type polyorganosiloxane et au moins un bloc d'un polyéther. Le bloc polyorganopolysiloxane peut être notamment un polydiméthylsiloxane ou bien encore un poly alkyl($C_2$-$C_{18}$) méthyl siloxane ; le bloc polyéther peut être un poly alkylène en $C_2$-$C_{18}$, en particulier polyoxyéthylène et/ou polyoxypropylène. En particulier, on peut utiliser les diméthicones copolyol ou des alkyl ($C_2$-$C_{18}$) diméthicones copolyol tels que ceux vendu sous la dénomination "Dow Corning 3225C" par la société Dow Corning, les lauryl méthicones tels que ceux vendu sous la dénomination "Dow Corning Q2-5200 par la société "Dow Corning".

Comme copolymères blocs greffés ou séquencés, on peut citer aussi ceux comprenant au moins un bloc résultant de la polymérisation d'au moins un monomère éthylénique, à une ou plusieurs liaisons éthyléniques éventuellement conjuguées, comme l'éthylène ou les diènes tels que le butadiène et l'isoprène, et d'au moins un bloc d'un polymère vinylique et mieux styrénique. Lorsque le monomère éthylénique comporte plusieurs liaisons éthyléniques éventuellement conjuguées, les insaturations éthyléniques résiduelles après la polymérisation sont généralement hydrogénées. Ainsi, de façon connue, la polymérisation de l'isoprène conduit, après hydrogénation, à la formation de bloc éthylène-propylène, et la polymérisation de butadiène conduit, après hydrogénation, à la formation de bloc éthylène-butylène. Parmi ces polymères, on peut citer les copolymères séquencés, notamment de type "dibloc" ou "tribloc" du type polystyrène/polyisoprène (SI), polystyrène/polybutadiène (SB) tels que ceux vendus sous le nom de 'LUVITOL HSB' par BASF, du type polystyrène/copoly(éthylène-propylène) (SEP) tels que ceux vendus sous le nom de 'Kraton' par Shell Chemical Co ou encore du type polystyrène/copoly(éthylène-butylène) (SEB). En particulier, on peut utiliser le Kraton G1650 (SEBS), le Kraton G1651 (SEBS), le Kraton G1652 (SEBS), le Kraton G1657X (SEBS), le Kraton G1701X (SEP), le Kraton G1702X (SEP), le Kraton G1726X (SEB), le Kraton D-1101 (SBS), le Kraton D-1102 (SBS), le Kraton D-1107 (SIS). Les polymères sont généralement appelés des copolymères de diènes hydrogénés ou non.

On peut aussi utiliser les Gelled Permethyl 99A-750, 99A-753-59 et 99A-753-58 (mélange de tribloc et de polymère en étoile), Versagel 5960 de chez Penreco (tribloc + polymère en étoile) ; OS129880, OS129881 et OS84383 de chez Lubrizol (copolymère styrène/méthacrylate).

Comme copolymères blocs greffés ou séquencés comprenant au moins un bloc résultant de la polymérisation d'au moins un monomère éthylénique à une ou plusieurs liaisons éthyléniques et d'au moins un bloc d'un polymère acrylique, on peut citer les copolymères bi- ou triséquencés poly(méthylacrylate de méthyle)/polyisobutylène ou les copolymères greffés à squelette poly(méthylacrylate de méthyle) et à greffons polyisobutylène.

Comme copolymères blocs greffés ou séquencés comprenant au moins un bloc résultant de la polymérisation d'au moins un monomère éthylénique à une ou plusieurs liaisons éthyléniques et d'au moins un bloc d'un polyéther tel qu'un polylkylène en $C_2$-$C_{18}$ (polyéthyléné et/ou polyoxypropyléné notamment), on peut citer les copolymères bi- ou triséquencés polyoxyéthylène/polybutadiène ou polyoxyéthylène/polyisobutylène.

Lorsqu'on utilise un polymère statistique en tant que stabilisant, on le choisit de manière à ce qu'il possède une quantité suffisante de groupements le rendant soluble dans le milieu de synthèse organique envisagé.

On peut ainsi employer des copolymères à base d'acrylates ou de méthacrylates d'alkyle issus d'alcools en $C_1$-$C_4$, et d'acrylates ou de méthacrylates d'alkyle issus d'alcools en $C_8$-$C_{30}$. On peut en particulier citer le copolymère méthacrylate de stéaryle/méthacrylate de méthyle.

Lorsque le milieu de synthèse est apolaire, il est préférable de choisir en tant que stabilisant, un polymère apportant une couverture des particules la plus complète possible, plusieurs chaînes de polymères-stabilisants venant alors s'adsorber sur une particule de polymère obtenu par polymérisation.

Dans ce cas, on préfère alors utiliser comme stabilisant, soit un polymère greffé, soit un polymère séquencé, de manière à avoir une meilleure activité interfaciale. En effet, les séquences ou greffons insolubles dans le solvant

de synthèse apportent une couverture plus volumineuse à la surface des particules.

Lorsque le solvant de synthèse liquide comprend au moins une huile de silicone, l'agent stabilisant est de préférence choisi dans le groupe constitué par les copolymères blocs greffés ou séquencés comprenant au moins un bloc de type polyorganosiloxane et au moins un bloc d'un polymère radicalaire ou d'un polyéther ou d'un polyester comme les blocs polyoxypropyléné et/ou oxyéthyléné.

Lorsque la phase organique liquide ne comprend pas d'huile de silicone, l'agent stabilisant est de préférence choisi dans le groupe constitué par :

- (a) les copolymères blocs greffés ou séquencés comprenant au moins un bloc de type polyorganosiloxane et au moins un bloc d'un polymère radicalaire ou d'un polyéther ou d'un polyester,
- (b) les copolymères d'acrylates ou de méthacrylates d'alkyle issus d'alcools en $C_1$-$C_4$, et d'acrylates ou de méthacrylates d'alkyle issus d'alcools en $C_8$-$C_{30}$,
- (c) les copolymères blocs greffés ou séquencés comprenant au moins un bloc résultant de la polymérisation d'au moins un monomère éthylénique, à liaisons éthyléniques conjuguées,

et au moins un bloc d'un polymère vinylique ou acrylique ou d'un polyéther ou d'un polyester, ou leurs mélanges.

De préférence, on utilise des polymères dibloc comme agent stabilisant.

***Plastifiant***

Lorsque le polymère présente une température de transition vitreuse trop élevée pour l'application souhaitée, on peut lui associer un plastifiant. Le plastifiant peut être choisi parmi les plastifiants usuellement utilisés dans le domaine d'application et notamment parmi les composés susceptibles d'être des solvants du polymère. On peut aussi utiliser des agents de coalescence afin d'aider la polymère à former un dépôt continu et homogène.

Les agents de coalescence ou plastifiants utilisables dans l'invention sont notamment ceux cités dans le document FR-A-2 782 917.

La composition selon l'invention peut comprendre au moins un ester d'au moins un acide carboxylique comprenant 1 à 7 atomes de carbones et d'un polyol comprenant au moins 4 groupes hydroxyles, ledit ester ayant une masse moléculaire inférieure à 5000 g/mol.

Le polyol peut être un mono- ou un polysaccharide comprenant un à 10 oses, de préférence de un à 4, de préférence encore un ou deux oses.

Le polyol selon l'invention est de préférence un disaccharide. Parmi les disaccharides, on peut citer le saccharose (alpha-D-glucopyranosyl-(1-2)-béta-D-fructofuranose), le lactose (béta-D-galactopyranosyl-(1-4)-béta-D-glucopyranose) et le maltose (alpha-D-glucopyranosyl-(1-4)-béta-D-glucopyranose).

Selon un mode de mise en oeuvre préféré, l'ester est le di-acétate-hexa-(2-méthyl-propanoate) de saccharose.

L'ester est de préférence liquide à température ambiante et pression atmosphérique. Il est avantageusement présent en une quantité comprise entre 0,1 et 25% en poids, de préférence entre 0,5 et 15% en poids, de préférence encore entre 3 et 15% en poids.

Le rapport massique entre les particules de polymère et l'ester d'acide et de polyol est avantageusement compris entre 0,5 à 100, de préférence entre 1 et 50, de préférence entre 1 et 10, de préférence encore entre 1 et 5.

**Phase grasse**

La phase grasse de la composition comprend avantageusement au moins une huile cosmétiquement ou dermatologiquement acceptable, et de façon générale physiologiquement acceptable, notamment choisie parmi les huiles d'origine minérale, végétale ou synthétique, carbonées, hydrocarbonées, fluorées et/ou siliconées, seules ou en mélange.

Par « huile », au sens de la demande, on entend tout milieux non aqueux liquide à température ambiante (25°C) et pression atmosphérique (760 mm de Hg).

La phase grasse totale de la composition peut représenter de 5 % à 90 % du poids total de la composition et de préférence de 20 à 85 %. Avantageusement, elle représente au moins 30 % du poids total de la composition.

Selon un mode de mise en oeuvre, la phase grasse est exempte ou contient moins de 50% en poids d'au moins une huile volatile par rapport au poids total de ladite phase grasse. De préférence, la phase grasse contient moins de 40%, de préférence m oins de 30%, de préférence moins de 20%, de préférence encore moins de 10% en poids d'au moins une huile volatile par rapport au poids total de ladite phase grasse.

***Huile apolaire ou peu polaire***

Selon un mode de réalisation, la phase grasse comprend au moins une huile apolaire ou peu polaire, laquelle représente au moins 5% en poids par rapport au poids total de la composition. En particulier, la ou les huiles de la phase grasse sont des huiles apolaires ou peu polaires hydrocarbonée(s) non volatiles, de préférence hydrocarbonées.
En particulier, les huiles apolaires ont un paramètre de solubilité $\delta_a = 0$.
On entend par huile polaire, une huile composée de composés chimiques comportant au moins un groupement polaire. Les "groupes polaires" sont bien connus de l'homme du métier ; il peut s'agir par exemple des groupes polaires ioniques ou non ioniques choisis parmi -COOH ; -OH ; oxyde d'éthylène; oxyde de propylène; $-PO_4$ ; -NHR ; $-NR_1R_2$ avec $R_1$, $R_2$ formant éventuellement un cycle et représentant un radical alkyle ou alkoxy linéaire ou ramifié en $C_1$ à $C_{20}$.
Les huiles peu polaires comprennent les huiles qui ont un paramètre moyen de solubilité à 25°C de : $0 < \delta_a < 5{,}0\ (J/cm^3)^{½}$.
Les huiles fortement polaires ont un paramètre moyen de solubilité $\delta_a$ selon l'espace de solubilité de Hansen, à 25°C, de : $\delta_a \geq 5{,}0\ (J/cm^3)^{½}$.
La définition et le calcul des paramètres de solubilité dans l'espace de solubilité tridimensionnel de HANSEN s ont d écrits d ans l'article d e C. M. H ANSEN : "The three dimensionnal solubility parameters" J. Paint Technol. 39, 105 (1967).
Selon cet espace de Hansen :

- $\delta_D$ caractérise les forces de dispersion de LONDON issues de la formation de dipôles induits lors des chocs moléculaires ;
- $\delta_p$ caractérise les forces d'interactions de DEBYE entre dipôles permanents ainsi que les forces d'interactions de KEESOM entre dipôles induits et dipôles permanents;
- $\delta_h$ caractérise les forces d'interactions spécifiques (type liaisons hydrogène, acide/base, donneur/accepteur, etc.) ;
- $\delta_a$ est déterminé par l'équation : $\delta_a = (\delta_p^2 + \delta_h^2)^{½}$
  Les paramètres $\delta_p$, $\delta_h$, $\delta_D$ et $\delta_a$ sont exprimés en $(J/cm^3)^{½}$.
  Lorsque la phase huileuse est un mélange de différentes huiles, les paramètres de solubilité du mélange sont déterminés à partir de ceux des composés pris séparément, selon les relations suivantes :

$$\delta_{Dmel} = \sum_i xi\ \delta_{Di}\ ; \qquad \delta_{pmel} = \sum_i xi\ \delta_{pi} \qquad \text{et} \qquad \delta_{hmel} = \sum_i xi\ \delta_{hi}$$

$$\delta_{amel} = (\delta^2_{pmel} + \delta^2_{hmel})^{1/2}$$

où xi représente la fraction volumique du composé i dans le mélange.

Il est à la portée de l'homme du métier de déterminer les quantités de chaque huile pour obtenir une phase huileuse satisfaisant aux critères souhaités.
L'huile apolaire ou peu polaire est de préférence hydrocarbonée
Par « huile hydrocarbonée », on entend une huile formée essentiellement, voire constituée, d'atomes de carbone et d'hydrogène, et éventuellement d'atomes d'oxygène, d'azote, et ne contenant pas d'atome de silicium ou de fluor. Elle peut contenir des groupes alcool, ester, éther, acide carboxylique, amine et/ou amide.
L'huile apolaire ou peu polaire est de préférence non volatile.
On entend par « huile non volatile », toute huile ayant une pression de vapeur à température ambiante et pression atmosphérique, non nulle inférieure à 0,02 mm de Hg et mieux inférieur à $10^{-3}$ mm de Hg.
L'huile apolaire ou peu polaire représente avantageusement au moins 5% en poids du poids total de la composition. L'huile apolaire ou peu polaire représente avantageusement 5% à 80% en poids du poids total de la composition, de préférence de 10% à 60% en poids, de préférence encore de 10% à 40% en poids par rapport au poids total de la composition.
L'huile apolaire ou peu polaire hydrocarbonée, représente de préférence de 10% à 40% en poids, de préférence de 15 à 30% en poids par rapport au poids total de la composition.
De préférence, l'huile apolaire ou peu polaire est une hydrocarbonée apolaire non volatile.
Selon un mode de réalisation l'huile hydrocarbonée apolaire est dépourvue d'hétéroatome(s). On entend par hétéroatome, un atome différent du carbone ou de l'hydrogène.
Selon un mode de réalisation, l'huile apolaire hydrocarbonée non volatile est avantageusement choisie parmi les alcanes saturés, linéaires ou ramifiés.

L'huile hydrocarbonée apolaire ou peu polaire non volatile peut être choisie parmi les huiles hydrocarbonées dont la masse moléculaire est comprise entre 300 et 900 g/mol, de préférence entre 350 et 800 g/mol.

Selon un mode de réalisation, l'huile apolaire hydrocarbonée non volatile est choisie parmi les hydrocarbures linéaires ou ramifiés tels que les huiles de paraffine, de vaseline et de naphtalène, le polyisobutène hydrogéné, l'isoeicosane, le squalane, les copolymères décène/butène et leurs mélanges.

Selon un mode de réalisation, la phase grasse comprend de 30 à 70% en poids d'huile(s) hydrocarbonée(s) non volatile(s) apolaire(s) rapport au poids de la phase grasse, de préférence de 40 à 60% en poids par rapport au poids de la phase grasse.

Comme autre exemples d'huile apolaires ou peu polaires non volatiles, on peut citer :

- les huiles hydrocarbonées d'origine animale comme le squalène ;
- les huiles hydrocarbonées végétales telles que les triglycérides liquides d'acides gras d'au moins 10 atomes de carbone;
- les esters et les éthers de synthèse notamment d'acides gras comme les huiles de formule $R_1(CO)_xOR_2$ dans laquelle $R_1$ représente le reste d'un acide comportant de 2 à 29 atomes de carbone avec x valant 0 ou 1 et $R_2$ représente une chaîne hydrocarbonée contenant de 3 à 30 atomes de carbone comme par exemple l'acétyl-citrate de tributyle, l'érucate d'oléyle, le béhénate d'octyl-2 dodécyle, le citrate de tri-iso-arachidyle, le stéaroyl-stéarate d'isocétyle ou d'octyldodécanyle, l'acétate de n-propyle, le tri-méllitate de tri-décyle, le dodécane di-oléate ou le stéarate de di-iso-cétyle, le propionate d'arachidyle, le dibutyl phtalate, le carbonate de propylène, le pentanoate d'octyldodécyle ; les esters de polyol comme la vitamine F, l'isostéarate de sorbitane, le triisostéarate de glycérine ou de diglycérine ;
- leurs mélanges.

La phase grasse de la composition peut également comprendre une huile siliconée non volatile choisie parmi

- les polydiméthylsiloxanes (PDMS), comportant éventuellement une chaîne alkyle en $C_3$-$C_{40}$, ou alcoxy en $C_3$-$C_{40}$, ou un radical phénylé ; les polydiméthylsiloxanes comportant des radicaux phénylés peuvent être choisies parmi les phényltriméthicones ;
- les polyalkylméthylsiloxanes, éventuellement fluorées comme les polyméthyltrifluoro-propyldiméthylsiloxanes,
- les polyalkylméthylsiloxanes substituées par des groupements fonctionnels tels que des groupements hydroxyle, thiol et/ou amine;
- les polysiloxanes modifiés par des acides gras, des alcools gras ou des polyoxyalkylènes.
  De préférence, la phase grasse de la composition contient moins de 10% d'une ou de plusieurs huiles siliconées. De préférence la phase grasse contient moins de 5%, moins de 3%, voir moins de 1 % d'une ou de plusieurs huiles siliconées.

*Huile fortement polaire non volatile*

La phase grasse peut contenir, outre l'huile apolaire ou peu polaire telle que décrite précédemment, une huile non volatile fortement polaire choisie parmi les esters d'acide gras de 7 à 29 atomes de carbone comme le malate de diisostéaryle, le palmitate d'isopropyle, l'adipate de diisopropyle, les triglycérides des acides caprylique/caprique comme ceux vendus par la société Stearineries Dubois ou ceux vendus sous les dénominations Miglyol 810, 812 et 818 par la société Dynamit Nobel, l'huile de beurre de karité, le myristate d'isopropyle, le stéarate de butyle, le laurate d'hexyle, l'adipate de diisopropyle, l'isononate d'isononyle, le laurate de 2-hexyl-décyle, le palmitate de 2-octyl-décyle, le myristate ou le lactate de 2-octyl-dodécyle, le succinate de 2-diéthyl-hexyle, le palmitate d'éthyl-2-hexyle, le stéarate d'octyl-2-dodécyle, l'huile de ricin ; les esters d'acide lanolique, d'acide laurique, d'acide stéarique ; les alcools gras supérieurs (de 7 à 29 atomes de carbone) tels que l'alcool stéarylique, l'alcool linoléique ou linolénique, l'alcool isostéarique, l'octyl-2-dodécanol, le décanol, le dodécanol, l'octadécanol ou l'alcool oléique ; les acides gras supérieurs (de 7 à 29 atomes de carbone) tels que l'acide myristique, l'acide palmitique, l'acide stéarique, l'acide béhénique, l'acide oléique, l'acide linoléique, l'acide linolénique ou l'acide isostéarique ; leurs mélanges.

Ces huiles fortement polaires non volatiles peuvent représenter de 0,1 à 10 % du poids total de la composition et mieux de 1 à 5 %.

*Huile volatile de la phase grasse*

On peut inclure une ou plusieurs huiles volatiles dans la phase grasse de la composition selon l'invention, à condition qu'elle représente moins de 50% en poids de la phase grasse. Ces huiles peuvent être des huiles hydrocarbonées ou des huiles siliconées comportant éventuellement des groupements alkyle ou alkoxy pendants ou en bout de chaîne siliconée.

On entend par « huile volatile », toute huile ayant une pression de vapeur à température ambiante et pression

atmosphérique, supérieure à 0,02 mm de Hg.

Comme huile de silicone volatile utilisable dans l'invention, on peut citer les silicones linéaires ou cycliques ayant une viscosité à température ambiante inférieure à 8 cSt et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl-cyclotétrasiloxane, le décaméthyl-cyclopentasiloxane, le dodécaméthylcyclohexasiloxane, l'heptaméthylhexyltrisiloxane, l'heptaméthyloctyltrisiloxane, l'octaméthyltrisiloxane, le décaméthyltétrasiloxane et leurs mélanges.

Comme huile volatile utilisable dans l'invention, on préfère notamment les huiles isoalcanes (appelées aussi isoparaffines) en $C_8$-$C_{16}$ comme l'isododécane, l'isodécane, l'isohexadécane et par exemple les huiles vendus sous les noms commerciaux d'ISOPAR, de PERMETHYL et notamment l'isododécane (PERMETHYL 99 A).

De préférence, la phase grasse de la composition contient moins de 10% d'une ou de plusieurs huiles volatiles. De préférence la phase grasse contient moins de 5%, moins de 3%, voir moins de 1 % d'une ou de plusieurs huiles volatiles.

*Huile de masse moléculaire élevée*

Selon un mode de réalisation, la phase grasse peut contenir, outre l'huile apolaire ou peu polaire au moins une huile de masse moléculaire élevée, par exemple comprise entre 650 à 10000 g/mol.

La composition selon l'invention contient avantageusement de 2 à 30 %, de préférence de 5 à 25%, de 5 à 15% d'au moins une huile de masse molaire allant de 650 à 10000 g/mol, et de préférence allant de 750 et 7500 g/mol.

L'huile de masse molaire allant de 650 à 10000 g/mol peut être choisie parmi :

- les polybutylènes tels que L'INDOPOL H-100 (de masse molaire ou MM=965 g/mol), L'INDOPOL H-300 (MM=1340 g/mol), L'INDOPOL H-1500 (MM=2160g/mol) commercialisés ou fabriqués par la société AMOCO,

- les polyisobutylènes hydrogénés tels que le PANALANE H-300 E commercialisés ou fabriqué par la société AMOCO (M =1340 g/mol), le VISEAL 20000 commercialisé ou fabriqué par la société SYNTEAL (MM=6000 g/mol), le REWOPAL PIB 1000 commercialisé ou fabriqué par la société WITCO (MM=1000 g/mol),

- les polydécènes et les polydécènes hydrogénés tels que : le PURESYN 10 (MM=723 g/mol), le PURESYN 150 (MM=9200 g/mol) commercialisé ou fabriqués par la société MOBIL CHEMICALS,

- les copolymères de la vinylpyrrolidone tels que : le copolymère vinylpyrrolidone/1-héxadécène, ANTARON V-216 commercialisé ou fabriqué par la société ISP (MM=7300 g/mol),

- les esters tels que :

    a) les esters d'acides gras linéaires ayant un nombre total de carbone allant de 35 à 70 comme le tétrapélargonate de pentaérythrityle (MM=697,05 g/mol),
    b) les esters hydroxylés tels que le triisostéarate de polyglycérol-2 (MM=965,58 g/mol),
    c) les esters aromatiques tels que le tridécyl trimellitate (MM=757,19 g/mol),
    d) les esters d'alcool gras ou d'acides gras ramifiés en $C_{24}$-$C_{28}$ tels que ceux décrits dans la demande EP-A-0 955 039, et notamment le citrate de triisoarachidyle (MM=1033,76 g/mol), le t étraisononanoate de pentaérythrityle (MM=697,05g/mol), le triisostéarate de glycéryle (MM=891.51 g /mol), le tridécyl-2 tétradécanoate de glycéryle (MM=1143,98 g/mol), le tétraisostéarate de pentaérythrityle (MM=1202,02 g/mol), le tétraisostéarate de polyglycéryle -2 (MM=1232,04 g/mol) ou encore le tétra décyl -2 tétradécanoate de pentaérythrityle (MM=1538,66 g/mol),
    e) les esters et polyesters de dimère diol, tels que les esters de dimère diol et d'acide gras, et les esters de dimère diols et de diacide.

    Les esters de dimère diol et d'acide mono-carboxylique peuvent être obtenus à partir d'acide mono-carboxylique comprenant de 4 à 34 atomes de carbone, notamment de 10 à 32 atomes de carbone, lesquels acides sont linéaires, ramifiés, saturés ou insaturés.

    A titre illustratif des exemples d'acide mono-carboxylique convenant à l'invention, on peut notamment citer les acides gras.

    Les esters de dimère diol et d'acide dicarboxylique peuvent être obtenus à partir d'un dimère diacide dérivé en particulier de la dimérisation d'un acide gras insaturé notamment en $C_8$ à $C_{34}$, notamment en $C_{12}$ à $C_{22}$, en particulier en $C_{16}$ à $C_{20}$, et plus particulièrement en $C_{18}$.

    Selon une variante particulière, il s'agit plus particulièrement du dimère diacide dont dérive également le dimère diol à estérifier.

Les esters de dimère diol peuvent être obtenus à partir d'un dimère diol produit par hydrogénation catalytique d'un dimère diacide tel que décrit précédemment, par exemple le diacide dilinoléique hydrogéné.

A titre illustratif des esters de dimère diol, on peut notamment citer les esters de diacides dilinoléiques et de dimères diols dilinoléiques commercialisés par la société NIPPON FINE CHEMICAL sous la dénomination commerciale LUSPLAN DD-DA5® et DD-DA7®.

- les huiles siliconées telles que les silicones phénylées comme la BELSIL PDM 1000 de la société WACKER (MM=9000 g/mol),
- les huiles d'origine végétale telles que l'huile de sésame (820,6 g/mol),
- et leurs mélanges.

*Cire, Pâteux*

La composition selon l'invention peut comprendre au moins une cire.

Une cire, au sens de la présente invention, est un composé gras lipophile, solide à température ambiante (25°C), à changement d'état solide/liquide réversible, ayant une température de fusion supérieure ou égal à 30°C, et présentant à l'état solide une organisation cristalline anisotrope. La taille des cristaux est telle que les cristaux diffractent et/ou diffusent la lumière, conférant à la composition un aspect trouble, plus ou moins opaque. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange.

Au sens de l'invention, la température de fusion de la cire correspond à la température du pic le plus endothermique observé en analyse thermique (DSC) telle que décrite dans la norme ISO 11357-3; 1999.
Le point de fusion de la cire peut être mesuré à l'aide d'un calorimètre à balayage différentiel (DSC), par exemple le calorimètre vendu sous la dénomination MDSC 2920 par la société TA Instruments.
Le protocole de mesure est par exemple le suivant :

> Un échantillon de 5 mg de cire disposé dans un creuset est soumis à une première montée en température allant de -20 °C à 100 °C, à la vitesse de chauffe de 10 °C/minute, puis est refroidi de 100 °C à -20 °C à une vitesse de refroidissement de 10 °C/minute et enfin soumis à une deuxième montée en température allant de -20 °C à 100 °C à une vitesse de chauffe de 5 °C/minute. Pendant la deuxième montée en température, on mesure la variation de la différence de puissance absorbée par le creuset vide et par le creuset contenant l'échantillon de cire en fonction de la température. Le point de fusion du composé est la valeur de la température correspondant au sommet du pic de la courbe représentant la variation de la différence de puissance absorbée en fonction de la température.

On entend par cire apolaire, une cire apolaire hydrocarbonée ou siliconée.

Les cires peuvent être hydrocarbonées, fluorées et/ou siliconées et être d'origine végétale, minérale, animale et/ou synthétique. En particulier, les cires présentent une température de fusion supérieure à 45 °C.
Comme cire utilisable dans la composition de l'invention, on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxy-diméticone ayant de 16 à 45 atomes de carbone.

La composition selon l'invention peut contenir une cire apolaire telle qu'une cire apolaire hydrocarbonée ou siliconée.

La composition contient avantageusement une cire hydrocarbonée apolaire. On entend par « cire hydrocarbonée apolaire » une cire contenant au moins 95% en poids de composés chimiques dépourvus de groupements polaires. Les "groupes polaires" sont bien connus de l'homme du métier; il peut s'agir par exemple de groupes polaires ioniques ou non ioniques choisis parmi -COOH ; -OH ; oxyde d'éthylène; oxyde de propylène; $-PO_4$ ; -NHR ; $-NR_1R_2$ avec $R_1$, $R_2$ formant éventuellement un cycle et représentant un radical alkyle ou alkoxy linéaire ou ramifié en $C_1$ à $C_{20}$.

Selon un mode de réalisation la cire hydrocarbonée contient au moins 95% en poids de composés dépourvus d'hétéroatomes. On entend par hétéroatome, un atome différent du carbone ou de l'hydrogène.

Selon un mode de réalisation, la cire hydrocarbonée apolaire contient au moins 95% en poids de composés chimiques constitués de carbone et d'hydrogène. Ces composés chimiques sont avantageusement choisis parmi les alcanes saturés, linéaires ou ramifiés.

Selon un mode de réalisation, la cire apolaire est choisie parmi les cires hydrocarbonées linéaires.

Les cires hydrocarbonées linéaires incluent les polymères et copolymères de l'éthylène, les cires de paraffine linéaires et les cires de Fischer Tropsch.

A titre illustratif et non limitatif de cires hydrocarbonées, on peut plus particulièrement citer les cires de Fischer-Tropsch, également appelées cires de polyméthylène. Elles répondent à la formule CnH2n+2.
Selon un mode de mise en oeuvre particulier de l'invention, la cire selon l'invention est une cire de polyméthylène, par exemple la cire Cirebelle 505®, fabriquée par la société SASOL, dont le point de fusion est égal à 40 °C.
La cire apolaire peut être une cire de type silicone polyoxyalkylénée c'est-à-dire une silicone comportant au moins un groupement oxyalkyléné de type $(-C_xH_{2x}O)_a$ dans lequel x peut varier de 2 à 6 et a est supérieur à ou égal à 2.
Les silicones oxyalkylénées susceptibles de convenir à l'invention peuvent être choisies parmi les composés de formules générales (I), (II), (III), (IV) ou (V) :

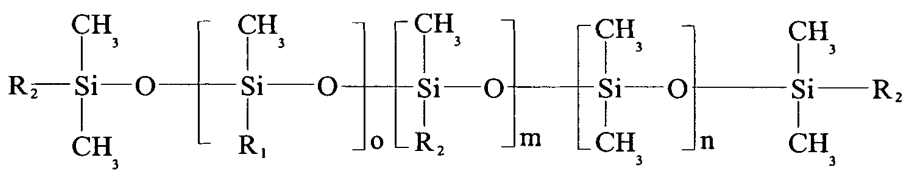

(I)

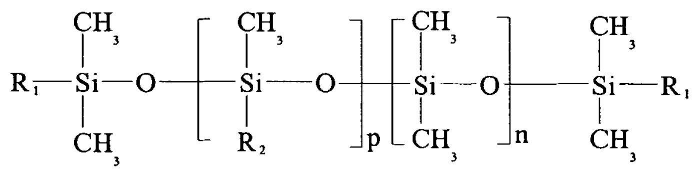

(II)

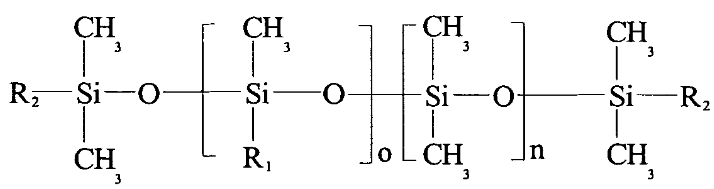

(III)

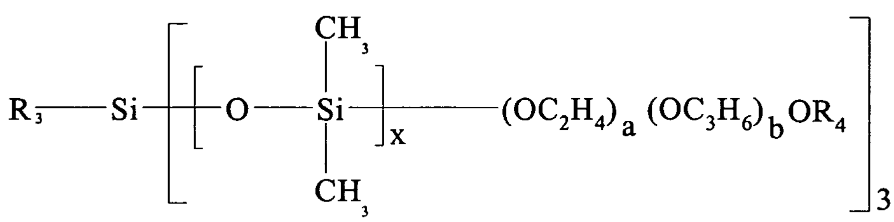

(IV)

formules (I), (II), (III) et (IV) dans lesquelles :

- $R_1$, identique ou différent, représente un radical alkyle, linéaire ou ramifié, en $C_1$-$C_{30}$ ou phényle,
- $R_2$, identique ou différent, représente un radical $C_cH_{2c}$-O-$(C_2H_4O)_a(C_3H_6O)_b$-$R_5$ ou un radical -$C_cH_{2c}$-O-$(C_4H_8O)_a$-$R_5$,
- $R_3$ et $R_4$, identiques ou différents, désignent un radical alkyle, linéaire ou ramifié, en $C_1$ à $C_{12}$ et de préférence le radical méthyle,
- $R_5$, identique ou différent, est choisi parmi un atome d'hydrogène, un radical alkyle, linéaire ou ramifié, de 1 à 12 atomes de carbone, un radical alcoxy, linéaire

ou ramifié, de 1 à 6 atomes de carbone, un radical acyle, linéaire ou ramifié, de 2 à 30 atomes de carbone, un radical hydroxyle, aminoalcoxy en $C_1$-$C_6$ éventuellement substitué sur l'amine, aminoacyle en $C_2$-$C_6$ éventuellement substitué sur l'amine, aminoalkyle éventuellement substitué sur l'amine et sur la chaîne alkyle, carboxyacyle en $C_2$-$C_{30}$, un groupement éventuellement substitué par un ou deux radicaux aminoalkyle substitués, -NHCO$(CH_2)_d$OH, un groupement phosphate, -M, identique ou différent, désigne un atome d'hydrogène, Na, K, Li, $NH_4$ ou une amine organique,

- $R_7$ désigne un atome d'hydrogène,
- d varie de 1 à 10,
- m varie de 0 à 20,
- n varie de 0 à 500,
- o varie de 0 à 20,
- p varie de 1 à 50,
- a varie de 0 à 50,
- b varie de 0 à 50,
- a + b est supérieur ou égal à 2,
- c varie de 0 à 4,
- x varie de 1 à 100.

De telles silicones sont par exemple décrites dans les brevets US-A-5 070 171, US-A-5149765, US-A-5093452 et US-A-5091493.

Conviennent tout particulièrement, les silicones de formule (III) dans laquelle $R_2$, identique ou différent, représente un radical $C_cH_{2c}$-O-$(C_2H_4O)_a(C_3H_6O)_b$-$R_5$, avec $R_5$, a, b et c étant définis comme précédemment. Dans ce mode de mise en oeuvre, b et c sont de préférence égaux à 0 et a est compris entre 1 et 50, de préférence entre 5 e t 30, de préférence encore entre 10 et 20.

La cire apolaire de bas point de fusion est avantageusement présente en une quantité comprise entre 1 et 30%, mieux encore entre 3 et 20% en poids par rapport au poids total de la composition.

Le rapport massique entre les particules de polymère et la cire apolaire de bas point de fusion est avantageusement compris entre 0,5 à 100, de préférence entre 1 et 50, de préférence entre 1 et 20, de préférence encore entre 3 et 15.

La composition selon l'invention peut, de plus comprendre au moins une cire additionnelle différente de la cire apolaire de bas point de fusion décrite précédemment.

La cire additionnelle a une température de fusion supérieure ou égal à 65°C. Elle peut être choisie parmi la cire d'abeilles, la cire de Carnauba ou de Candellila, la paraffine, les cires microcristallines, la cérésine ou l'ozokérite ; les cires de polyéthylène, les cires de Fischer Tropsch ; et leurs mélanges.

Selon un mode réalisation, la cire apolaire dont le point de fusion est inférieur à 65°C (a) et la cire dont la température de fusion est supérieure ou égal à 65°C (b) sont dans une proportion massique (a)/(b) comprise entre 30/70 et 55/45, de préférence entre environ 40/60 et environ 45/55.

La quantité de toutes les cire(s) contenues dans la composition va avantageusement de 15% à 35%, de préférence de 20% à 30% % en poids du poids total de la composition.

La cire additionnelle est de préférence une cire apolaire de point de fusion supérieur à 65°C, comme par exemple une cire microcristalline, une cire de polyéthylène, une cire de paraffine ou un de leurs mélanges.

La composition selon l'invention peut également contenir un composé pâteux.

*Milieu de synthèse des particules de polymère*

Selon un mode de réalisation particulièrement préféré, la phase grasse de la composition contient au moins une huile qui est le ou un des solvants organiques ayant servi de milieu de polymérisation des particules de polymère telles que décrites précédemment.

La dispersion de polymère peut être fabriquée comme décrit dans le document EP-A-749747.

On prépare un mélange comprenant les monomères initiaux ainsi qu'un amorceur radicalaire. Ce mélange est dissous dans un solvant appelé, dans la suite de la présente description, "solvant de synthèse".

On choisit un solvant de synthèse tel que les monomères initiaux, et l'amorceur radicalaire, y sont solubles, et les particules de polymère obtenues y sont insolubles afin qu'elles y précipitent lors de leur formation. En particulier, on peut choisir le solvant de synthèse parmi les alcanes tels que l'heptane, l'isododécane ou le cyclohexane. On peut effectuer la polymérisation des particules de polymère dans un solvant de synthèse tel que décrit précédemment, puis ajouter l'huile hydrocarbonée non volatile décrite précédemment et distiller sélectivement le solvant de synthèse, à la condition que l'huile hydrocarbonée non volatile soit miscible avec ledit solvant de synthèse.

Les monomères sont de préférence présents dans le solvant de synthèse, avant polymérisation, à raison de 5-20% en poids du mélange réactionnel. La totalité des monomères peut être présente dans le solvant avant le début de la réaction, ou une partie des monomères peut être ajoutée au fur et à mesure de l'évolution de la réaction de polymérisation.

L'amorceur radicalaire peut être notamment l'azo-bis-isobutyronitrile ou le tertiobutylperoxy-2-éthyl hexanoate.

**Milieu hydrophile**

La composition selon l'invention peut comprendre, un milieu hydrophile comprenant de l'eau ou un mélange d'eau et de solvant(s) organique(s) hydrophile(s) comme les alcools et notamment les monoalcools inférieurs linéaires ou ramifiés ayant de 2 à 5 atomes de carbone comme l'éthanol, l'isopropanol ou le n-propanol, et les polyols comme la glycérine, la diglycérine, le propylène glycol, le sorbitol, le penthylène glycol, et les polyéthylène glycols, ou bien encore des éthers en $C_2$ et des aldéhydes en $C_2$-$C_4$ hydrophiles.
L'eau ou le mélange d'eau et de solvants organiques hydrophiles peut être présent dans la composition selon l'invention en une teneur allant de 0,1 % à 99 % en poids, par rapport au poids total de la composition, et de préférence de 10 % à 80 % en poids.

**Polymère semi-cristallin**

Par "polymères", on entend au sens de l'invention des composés comportant au moins 2 motifs de répétition, de préférence au moins 3 motifs de répétition et plus spécialement au moins 10 motifs répétitifs.
Par "polymère semi-cristallin", on entend au sens de l'invention, des polymères comportant une partie cristallisable et une partie amorphe dans le squelette et présentant une température de changement de phase réversible du premier ordre, en particulier de fusion (transition solide-liquide). La partie cristallisable est soit une chaîne latérale (ou chaîne pendante), soit une séquence dans le squelette.
Lorsque la partie cristallisable du polymère semi-cristallin est une séquence du squelette polymérique, cette séquence cristallisable est de nature chimique différente de celle des séquences amorphes; le polymère semi-cristallin est dans ce cas un copolymère séquencé par exemple du type dibloc, tribloc ou multibloc. Lorsque la partie cristallisable est une chaîne pendante au squelette, le polymère semi cristallin peut être un homopolymère ou un copolymère.
Par "composé organique" ou "à structure organique", on entend des composés contenant des atomes de carbone et des atomes d'hydrogène et éventuellement des hétéroatomes comme S, O, N, P seuls ou en association.
La température de fusion du polymère semi-cristallin est de préférence inférieure à 150°C.
La température de fusion du polymère semi-cristallin est de préférence supérieure ou égale à 30°C et inférieure à 100°C. De préférence encore, la température de fusion du polymère semi-cristallin est de préférence supérieure ou égale à 30°C et inférieure à 60°C.
Le ou les polymères semi-cristallins selon l'invention servant sont des solides à température ambiante (25°C) et pression atmosphérique (760 mm de Hg), dont la température de fusion est supérieure ou égale à 30°C. Les valeurs de point de fusion correspondent au point de fusion mesuré à l'aide d'un calorimètre à balayage différentiel (D. S. C), tel que le calorimètre vendu sous la dénomination DSC 30 par la société METTLER, avec une montée en température de 5 ou 10°C par minute. (Le point de fusion considéré est le point correspondant à la température du pic le plus endotherme du thermogramme).
Le ou les polymères semi-cristallins selon l'invention ont de préférence une température de fusion supérieure à la température du support kératinique destiné à recevoir ladite composition, en particulier la peau ou les lèvres.
Le ou les polymères semi-cristallins selon l'invention peuvent être capables de structurer seuls ou en mélange, la composition sans ajout de tensioactif particulier, ni de charge, ni de cire.
Selon l'invention les polymères semi-cristallins sont avantageusement solubles dans la phase grasse, notamment à au moins 1 % en poids, à une température supérieure à leur température de fusion. En dehors des chaînes ou séquences cristallisables, les séquences des polymères sont amorphes.
Par "chaîne ou séquence cristallisable", on entend au sens de l'invention une chaîne ou séquence qui si elle était seule passerait de l'état amorphe à l'état cristallin, de façon réversible, selon qu'on est au-dessus ou en dessous de la température de fusion. Une chaîne au sens de l'invention est un groupement d'atomes, pendant ou latéral par rapport au squelette du polymère. Une séquence est un groupement d'atomes appartenant au squelette, groupement constituant un des motifs répétitif du polymère.
De préférence, le squelette polymérique des polymères semi-cristallins est soluble dans la phase grasse.
De préférence, les séquences ou chaînes cristallisables des polymères semi-cristallins représentent au moins 30 % du poids total de chaque polymère et mieux au moins 40 %. Les polymères semi-cristallins à chaînes latérales cristallisables sont des homo ou des copolymères. Les polymères semi-cristallins de l'invention à séquences cristallisables sont des copolymères, séquencés ou multiséquencés. Ils peuvent être obtenus par polymérisation de monomère à double liaisons réactives (ou éthyléniques) ou par polycondensation. Lorsque les polymères de l'invention sont des polymères à chaînes latérales cristallisables, ces derniers sont avantageusement sous forme aléatoire ou statistique.
De préférence, les polymères semi-cristallins de l'invention sont d'origine synthétique. Selon un mode de réalisation de l'invention, les polymères semi-cristallins de l'invention ne comportent pas de squelette polysaccharidique.

Les polymères semi-cristallins utilisables dans l'invention peuvent être choisis en particulier parmi:

- les copolymères séquencés de polyoléfines à cristallisation contrôlée, dont les monomères sont décrits dans EP-A-0 951 897.
- les polycondensats et notamment de type polyester, aliphatique ou aromatique ou aliphatique/aromatique,
- les homo- ou co-polymères portant au moins une chaîne latérale cristallisable et les homo- ou co-polymères portant dans le squelette au moins une séquence cristallisable, comme ceux décrits dans le document US-A-5 156 911,
- les homo- ou co-polymères portant au moins une chaîne latérale cristallisable en particulier à groupement(s) fluoré (s), tels que décrits dans le document WO-A-01/19333,
- et leurs mélanges.

Dans les deux derniers cas, la ou les chaînes latérales ou séquences cristallisables sont hydrophobes.

*A) Polymères semi-cristallins à chaînes latérales cristallisables*

On peut citer en particulier ceux définis dans les documents US-A-5 156 911 et WO-A-01/19333.

. Ce sont des homopolymères ou copolymères comportant de 50 à 100 % en poids de motifs résultant de la polymérisation de un ou plusieurs monomères porteurs de chaîne latérale hydrophobe cristallisable.

. Ces homo- ou co-polymères sont de toute nature du moment qu'ils présentent les conditions indiquées ci-après avec en particulier la caractéristique d'être solubles ou dispersables dans la phase grasse, par chauffage au-dessus de leur température de fusion Pf. Ils peuvent résulter :

  - de la polymérisation notamment radicalaire d'un ou plusieurs monomères à double(s) liaison(s) réactive(s) ou éthyléniques vis-à-vis d'une polymérisation, à savoir à groupe vinylique, (méth)acrylique ou allylique.
  - de la polycondensation d'un ou plusieurs monomères porteurs de groupes co-réactifs (acide carboxylique ou sulfonique, alcool, amine ou isocyanate), comme par exemple les polyesters, les polyuréthanes, les polyéthers, les polyurées, les polyamides.

  a) D'une façon générale les motifs (chaînes ou séquences) cristallisables des polymères semi-cristallins selon l'invention, proviennent de monomère(s) à séquence(s) ou chaîne(s) cristallisable(s), utilisé(s) pour la fabrication des polymères semi-cristallins. Ces polymères sont choisis notamment parmi les homopolymères et copolymères résultant de la polymérisation d'au moins un monomère à chaîne(s) cristallisable(s) qui peut être représenté par la formule X :

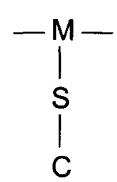

avec M représentant un atome du squelette polymérique
S représentant un espaceur
C représentant un groupe cristallisable

Les chaînes « -S-C » cristallisables peuvent être aliphatiques ou aromatiques, éventuellement fluorées ou perfluorées. « S » représente notamment un groupe $(CH_2)_n$ ou $(CH_2CH_2O)_n$ ou $(CH_2O)$, linéaire ou ramifié ou cyclique, avec n entier allant de 0 à 22. De préférence « S » est un groupe linéaire. De préférence, « S » et « C » sont différents.

Lorsque les chaînes cristallisables sont des chaînes aliphatiques hydrocarbonées, elles comportent des chaînes alkyle hydrocarbonées à au moins 11 atomes de carbone et au plus 40 atomes de carbone et mieux au plus 24 atomes de carbone. Il s'agit notamment de chaînes aliphatiques ou chaînes alkyle possédant au moins 12 atomes de carbone et de préférence, il s'agit de chaînes alkyle en $C_{14}$-$C_{24}$. de préférence en $C_{16}$-$C_{22}$ .Lorsqu'il s'agit de chaînes alkyle fluorées ou perfluorées, elles comportent au moins 11 atomes de carbone dont au moins 6 atomes de carbone sont fluorés.

Comme exemple d'homopolymères ou de copolymères semi-cristallins à chaîne(s) cristallisable(s), on peut citer ceux résultant de la polymérisation d'un ou plusieurs monomères suivants : les (méth)acrylates d'alkyle saturés

avec le groupe alkyle en $C_{14}$-$C_{24}$, les (méth)acrylates de perfluoroalkyle avec un groupe alkyle perfluoro en $C_{11}$-$C_{15}$, les N-alkyl (méth)acrylamides avec le groupe alkyle en $C_{14}$ à $C_{24}$ avec ou sans atome de fluor, les esters vinyliques à chaînes alkyle ou perfluoro (alkyle) avec le groupe alkyle en $C_{14}$ à $C_{24}$ (avec au moins 6 atomes de fluor pour une chaîne perfluoro alkyle), les éthers vinyliques à chaînes alkyle ou perfluoro (alkyle) avec le groupe alkyle en $C_{14}$ à $C_{24}$ et au moins 6 atomes de fluor pour une chaîne perfluoro alkyle, les alpha-oléfines en $C_{14}$ à $C_{24}$ comme par exemple l'octadécène, les para-alkyl styrènes avec un groupe alkyle comportant de 12 à 24 atomes de carbone, leurs mélanges.

Lorsque les polymères résultent d'une polycondensation, les chaînes cristallisables hydrocarbonées et/ou fluorées telles que définies ci-dessus, sont portées par un monomère qui peut être un diacide, un diol, une diamine, un di-isocyanate.

Lorsque les polymères objets de l'invention sont des copolymères, ils contiennent, en plus, de 0 à 50 % de groupes Y ou Z résultant de la copolymérisation :

α) de Y qui est un monomère polaire ou non polaire ou un mélange des deux :

- Lorsque Y est un monomère polaire, c'est soit un monomère porteur de groupes polyoxyalkylénés (notamment oxyéthyléné et/ou oxypropyléné), un (méth)acrylate d'hydroxyalkyle comme l'acrylate d'hydroxyéthyle, le (méth)acrylamide, un N-alkyl(méth)acrylamide, un NN-dialkyl(méth)acrylamide comme par exemple le NN-diisopropylacrylamide ou la N-vinyl-pyrolidone (NVP), le N-vinyl caprolactame, un monomère porteur d'au moins un groupe acide carboxylique comme les acides (méth)acryliques, crotonique, itaconique, maléique, fumarique ou porteur d'un groupe anhydride d'acide carboxylique comme l'anhydre maléique, et leurs mélanges.

- Lorsque Y est un monomère non polaire il peut être un ester du type (méth)acrylate d'alkyle linéaire ramifié ou cyclique, un ester vinylique, un alkyl vinyl éther, une alpha-oléfine, le styrène ou le styrène substitué par un groupe alkyle en $C_1$ à $C_{10}$, comme l'α-méthylstyrène, un macromonomère du type polyorganosiloxane à insaturation vinylique.

  Par "alkyle", on entend au sens au sens de l'invention un groupement saturé notamment en $C_8$ à $C_{24}$, sauf mention exprès.

β) de Z qui est un monomère polaire ou un mélange de monomères polaires. Dans ce cas, Z a la même définition que le "Y polaire" défini ci-dessus.

De préférence, les polymères semi-cristallins à chaîne latérale cristallisable sont des homopolymères d'alkyl (méth)acrylate o u d'alkyl(méth)acrylamide avec un groupe alkyle tel que défini ci-dessus, et notamment en $C_{14}$-$C_{24}$, des copolymères de ces monomères avec un monomère hydrophile de préférence de nature différente de l'acide (méth)acrylique comme la N-vinylpyrrolidone ou l'hydroxyéthyl (méth)acrylate et leurs mélanges.

De façon avantageuse, le ou les polymères semi-cristallins à chaîne latérale cristallisable ont une masse moléculaire moyenne en poids Mp allant de 5 000 à 1 000 000, de préférence de 10 000 à 800 000, préférentiellement de 15 000 à 500 000, de préférence encore de 100 000 à 200 000.

A titre d'exemple particulier de polymère semi-cristallin utilisable dans la composition selon l'invention, on peut citer les produits Intelimer® de la société Landec décrits dans la brochure "Intelimer® polymers", Landec IP22 (Rev. 4-97). Ces polymères sont sous forme solide à température ambiante (25°C). Ils sont porteurs de chaînes latérales cristallisables et présentent la formule X précédente.

Par exemple, on choisit le produit Intelimer® IPA 13-1 de la société Landec, qui est un polyacrylate de stéaryle de poids moléculaire d'environ 145 000 et dont la température de fusion est égale à 49°C.

Les polymères semi-cristallins peuvent être notamment ceux décrits dans les exemples 3, 4, 5, 7, 9 du brevet US-A-5 156 911 à groupement -COOH, résultant de la copolymérisation d'acide acrylique et d'alkyl(méth)acrylate en $C_5$ à $C_{16}$ de température de fusion allant de 20°C à 35°C et plus particulièrement de la copolymérisation :

- d'acide acrylique, d'hexadécylacrylate et d'isodécylacrylate dans un rapport 1/16/3,
- d'acide acrylique et de pentadécylacrylate dans un rapport 1/19,
- d'acide acrylique, d'hexadécylacrylate, éthylacrylate dans un rapport 2,5/76,5/20,
- d'acide acrylique, d'hexadécylacrylate et de méthylacrylate dans un rapport 5/85/10, d'acide acrylique, de polyoctadécylméthacrylate dans un rapport 2,5/97,5.

On peut aussi utiliser le polymère Structure « O » de National Starch tel que celui décrit dans le document US-A-5 736 125 de température de fusion de 44°C

Les polymères semi-cristallins peuvent être notamment les polymères semi-cristallins à chaînes pendantes

cristallisables comportant des groupements fluorés tels que décrits dans les exemples 1, 4, 6, 7 et 8 du document WO-A-01/19333.

On peut encore utiliser les polymères semi-cristallins obtenus par copolymérisation d'acrylate de stéaryle et d'acide acrylique ou de NVP tels que décrits dans le document US-A-5 519 063 ou EP-A- 550 745.

On peut aussi utiliser les polymères semi-cristallins obtenus par copolymérisation de l'acrylate de béhényle et de l'acide acrylique ou de NVP, tels que décrits dans les documents US-A-5 519 063 et EP-A- 055 0745 et plus spécialement ceux décrits dans les exemples 3 et 4, ci-après, de préparation de polymère.

*B) Les polymères portant dans le squelette au moins une séquence cristallisable*

Il s'agit encore de polymères solubles ou dispersables dans la phase grasse par chauffage au-dessus de leur point de fusion Pf. Ces polymères sont notamment des copolymères séquencés constitués d'au moins deux séquences de nature chimique différente dont l'une est cristallisable.

Le polymère portant dans le squelette au moins une séquence cristallisable peut être choisi parmi les copolymères séquencés d'oléfine ou de cyclooléfine à chaîne cristallisable comme ceux issus de la polymérisation séquencée de :

- cyclobutène, cyclohexène, cyclooctène, norbornène (c'est-à-dire bicyclo(2,2,1)heptène-2), 5-méthylnorbornène, 5-éthylnorbornène, 5,6-diméthylnorbornène, 5,5,6-triméthyl norbornène, 5-éthylidène-norbornène, 5-phényl-norbonène, 5-benzylnorbornène, 5-vinyl norbornène, 1,4,5,8-diméthano-1,2,3,4,4a,5,8a-octahydronaphtalène, dicyclopentadiène ou leurs mélanges, avec
- l'éthylène, le propylène, le 1-butène, le 3-méthyl-1-butène, le 1-hexène, le 4-méthyl-1-pentène, le 1-octène, le 1-décène, le 1-éicosène ou leurs mélanges, et en particulier les copoly(éthylène/norbornène) blocs et les terpolymères (éthylène/propylène/éthylidène-norbornène), blocs. On peut aussi utiliser ceux résultants de la copolymérisation séquencée d'au moins 2 $\alpha$-oléfines en $C_2$-$C_{16}$ et mieux en $C_2$-$C_{12}$ tels que ceux cités précédemment et en particulier les bipolymères séquencés d'éthylène et d'1-octène.

Le polymère portant dans le squelette au moins une séquence cristallisable peut être choisi parmi les copolymères présentant au moins une séquence cristallisable, le reste du copolymère étant amorphe (à température ambiante). Ces copolymères peuvent, en outre, présenter deux séquences cristallisables de nature chimique différente.

Les copolymères préférés sont ceux qui possèdent à la fois à température ambiante, une séquence cristallisable et une séquence amorphe à la fois hydrophobe et lipophile réparties séquentiellement. On peut citer par exemple les polymères possédant une des séquences cristallisables et une des séquences amorphes suivantes :

- Séquence cristallisable par nature de type polyester comme les poly(alkylène téréphtalate), ou de type polyoléfine comme les polyéthylènes

ou polypropylènes.

- Séquence amorphe et lipophile comme les polyoléfines ou copoly(oléfine)s amorphes telles que le poly(isobutylène), le polybutadiène hydrogéné, le poly(isoprène) hydrogéné.

Comme exemple de tels copolymères à séquence cristallisable et à séquence amorphe, on peut citer :

$\alpha$) les copolymères séquencés poly($\varepsilon$-caprolactone)-b-poly(butadiène), utilisés de préférence hydrogénés, tels que ceux décrits dans l'article D6 "Melting behavior of poly(-caprolactone)-block-polybutadiène copolymers" de S. Nojima, Macromolécules, 32, 3727-3734 (1999).

$\beta$) les copolymères séquencés poly(butylènetéréphtalate)-b-poly(isoprène) hydrogénés séquencés ou multiséquencés, cités dans l'article D7 "Study of morphological and mechanical properties of PP/PBT" de B. Boutevin et al., Polymer Bulletin, 34, 117-123 (1995).

$\gamma$) les copolymères séquencés poly(éthylène)-b-copoly(éthylène/propylène) cités dans les articles D8 "Morphology of semi-crystalline block copolymers of ethylene-(ethylene-alt-propylene)" de P. Rangarajan et al., Macromolecules, 26, 4640-4645 (1993) et D9 "Polymer agregates with crystalline cores: the system poly(ethylene)-poly(ethylene-propylene)" P. Richter et al., Macromolécules, 30, 1053-1068 (1997).

$\delta$) les copolymères séquencés poly(éthylène)-b-poly(éthyléthylène) cités dans l'article général D10 "Cristallization in block copolymers" de I.W. Hamley, Advances in Polymer Science, vol 148, 113-137 (1999).

C) *Polycondensats de type polyester, aliphatique ou aromatique ou aliphatique/aromatique*

Les polycondensats polyester peuvent être choisis parmi les polyesters aliphatiques. Leur masse moléculaire est de préférence supérieure ou égale à 200 et inférieure ou égal à 10000, et de préférence encore supérieure ou égale à 300 et inférieure ou égal à 5000, de préférence supérieure ou égale à 500 et supérieure ou égale à 2 000 g/mol.
Les polycondensats polyester sont en particulier choisis parmi les polycaprolactones. En particulier, les polycaprolactones peuvent être choisies parmi les homopolymères d'ε-caprolactones. L'homopolymérisation peut être initiée avec un diol, notamment un diol ayant de 2 à 10 atomes, tels que le diéthylène glycol, le 1,4-butanediol, le néopentyl glycol.
On peut utiliser par exemple les polycaprolactones, notamment celles commercialisées sous le nom de CAPA® 240 (point de fusion de 68°C et poids moléculaire de 4000), 223 (point de fusion de 48°C et poids moléculaire de 2000), 222 (point de fusion de 48°C et poids moléculaire de 2000), 217 (point de fusion de 44°C et poids moléculaire de 1250), 2125 (point de fusion de 45°C et poids moléculaire de 1250), 212 (point de fusion de 45°C et poids moléculaire de 1000), 210 (point de fusion de 38°C et poids moléculaire de 1000), 205 (point de fusion de 39°C et poids moléculaire de 830) par la société SOLVAY, PCL-300, PCL-700 par la société UNION CARBIDE.
On peut utiliser en particulier la CAPA® 2125 dont la température de fusion est comprise entre 35 et 45°C et dont la masse moléculaire est poids est égale à 1250.
Les polymères semi-cristallins de la composition de l'invention peuvent être ou non réticulés en partie du moment où le taux de réticulation ne gène pas leur dissolution ou dispersion dans la phase grasse par chauffage au-dessus de leur température de fusion. Il peut s'agir alors d'une réticulation chimique, par réaction avec un monomère multifonctionnel lors de la polymérisation. Il peut aussi s'agir d'une réticulation physique qui peut alors être due soit à l'établissement de liaisons type hydrogène ou dipolaire entre des groupes portés par le polymère comme par exemple les interactions dipolaires entre ionomères carboxylates, ces interactions étant en faible quantité et portées par le squelette du polymère ; soit à une séparation de phase entre les séquences cristallisables et les séquences amorphes, portées par le polymère.
De préférence, les polymères semi-cristallins de la composition selon l'invention sont non réticulés.
En pratique, la quantité totale de polymère(s) semi-cristallin(s) représente de 0,1 à 80 % du poids total de la composition et mieux de 0,5 à 40 % et encore mieux de 3 à 30 %. De préférence, il représente de 5 % à 25% en poids de la composition.

**Phase particulaire**

La composition selon l'invention peut contenir des pigments et/ou des charges.
Les pigments peuvent être blancs ou colorés, minéraux et/ou organiques, interférentiels ou non. On peut citer, parmi les pigments minéraux, le dioxyde de titane, éventuellement traité en surface, les oxydes de zirconium ou de cérium, ainsi que les oxydes de zinc, de fer (noir, jaune ou rouge ou de chrome, le violet de manganèse, le bleu outremer, l'hydrate de chrome et le bleu ferrique. Parmi les pigments organiques, on peut citer le noir de carbone, les pigments de type laques organiques de baryum, strontium, calcium ou aluminium dont celles soumises à une certification par la Food and Drug Administration (FDA) (exemple D&C ou FD&C) et ceux exempts de la certification FDA comme les laques à base de carmin de cochenille. Les pigments peuvent représenter de 0,1 à 50 % en matière active et notamment d e 0,5 à 35 % et mieux de 2 à 25 % du poids total de la composition.
Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth. Ils peuvent représenter de 0 à 25 % (en matière active) du poids total de la composition et mieux de 0,1 à 15 % (si présents). On peut ainsi utiliser des pigments à propriétés goniochromatiques, et/ou des pigments à effet métallique tel que décrit dans la demande déposée pour le numéro FR 0209246 dont le contenu est incorporé par référence dans la présente demande.
Les charges peuvent être minérales ou organiques, lamellaires ou sphériques. On peut citer le talc, le mica, la silice, le kaolin, les poudres de Nylon® (Orgasol® de chez Atochem), de poly-β-alanine et de polyéthylène, les poudres de polymères de tétrafluoroéthylène (Téflon®), la lauroyl-lysine, l'amidon, le nitrure de bore, les microsphères creuses telles que l'Expancel® (Nobel Industrie), le Polytrap® (Dow Corning) et les microbilles de résine de silicone (Tospearls® de Toshiba, par exemple), le carbonate de calcium précipité, le carbonate et l'hydro-carbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.
La composition selon l'invention contient de p référence des particules solides à température ambiante, dispersées dans le milieu physiologiquement acceptable introduites dans la composition sous forme d'une dispersion colloïdale, telle que décrite dans la demande WO 02/39961 dont le contenu est incorporé par référence dans la présente demande.

La composition peut contenir au m oins un agent dispersant. L'agent dispersant sert notamment à protéger les particules de charge ou de pigment dispersées contre leur agglomération ou floculation. La concentration en dispersant généralement utilisée pour stabiliser une dispersion colloïdale est de 0,3 à 5 mg/m$^2$, de préférence de 0,5 à 4 mg/m$^2$, d e surface d e particules de pigments et/ou de charges. Cet agent dispersant peut être un tensioactif, un oligomère, un polymère ou un mélange de plusieurs d'entre eux, portant une ou des fonctionnalités ayant une affinité forte pour la surface des particules à disperser. En particulier, ils peuvent s'accrocher physiquement ou chimiquement à la surface des pigments. Ces dispersants présentent, en outre, au moins un groupe fonctionnel compatible ou soluble dans le milieu continu. En particulier, on utilise les esters de l'acide hydroxy-12 stéarique en particulier et d'acide gras en $C_8$ à $C_{20}$ et de polyol comme le glycérol, la diglycérine, tel que le stéarate d'acide poly(12-hydroxystéarique) de poids moléculaire d'environ 750g/mole tel que celui vendu sous le nom de Solsperse 21 000 par la société Avecia, le polyglycéryl-2 dipolyhydroxystéarate (nom CTFA) vendu sous la référence Dehymyls PGPH par la société Henkel ou encore l'acide polyhydroxystéarique tel que celui vendu sous la référence Arlacel P100 par la société Uniqema et leurs mélanges.

Comme autre dispersant utilisable dans la composition de l'invention, on peut citer les dérivés ammonium quaternaire d'acides gras polycondensés comme le Solsperse 17000 vendu par la société Avecia, les mélanges de poly diméthylsiloxane/oxypropylène tels que ceux vendus par la société Dow Corning sous les références DC2-5185, DC2-5225 C.

L'acide polydihydroxystéarique et les esters de l'acide hydroxy-12-stéarique sont de préférence destinés à un milieu hydrocarboné ou fluoré, alors que les mélanges de diméthylsiloxane oxyéthylène/oxypropyléné sont de préférence destinés à un milieu siliconé.

La dispersion colloïdale est une suspension de particules de taille généralement micronique (<10 µm) dans un milieu continu. La fraction volumique de particules dans une dispersion concentrée est de 20 % à 40 %, de préférence supérieure à 30 %, ce qui correspond à une teneur pondérale pouvant aller jusqu'à 70 % selon la densité des particules.

**Additifs et galéniques**

La composition de l'invention peut, en outre, contenir un ou plusieurs actifs cosmétiques ou dermatologiques tels que ceux classiquement utilisés.

Comme actifs cosmétiques, dermatologiques, hygiéniques ou pharmaceutiques, utilisables dans la composition de l'invention, on peut citer les hydratants, vitamines, acides gras essentiels, sphingolipides, filtres solaires. Ces actifs sont utilisés en quantité habituelle pour l'homme de l'art et notamment à des concentrations de 0 à 20 % et notamment de 0,001 à 15 % du poids total de la composition.

La composition peut comprendre, en outre, tout autre additif usuellement utilisé dans de telles compositions, tel que de l'eau, des gélifiants, , des colorants hydrosolubles, des antioxydants, des parfums, des conservateurs et des huiles essentielles.

Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

Dans un mode de réalisation particulier de l'invention, les compositions selon l'invention peuvent être préparées de manière usuelle par l'homme du métier. Elles peuvent se présenter sous forme d'un produit coulé et par exemple sous la forme d'un stick ou bâton, ou sous la forme de coupelle utilisable par contact direct ou à l'éponge. En particulier, elles trouvent une application en tant que fond de teint coulé, fard à joues ou à paupières coulé, rouge à lèvres, base ou baume de soin pour les lèvres, produit anti-cernes. Elles peuvent aussi se présenter sous forme d'une pâte souple ou encore de gel, de crème plus ou moins fluide, ou de liquide conditionnés dans d'un tube. Elles peuvent alors constituées des fonds de teint ou des rouges à lèvres, des produits solaires ou de coloration de la peau.

La composition de l'invention est avantageusement anhydre et contiennent dans ce cas moins de 5 % d'eau par rapport au poids total de la composition.

Ces compositions à application topique peuvent constituer notamment une composition cosmétique, dermatologique, hygiénique ou pharmaceutique de protection, de traitement ou de soin pour le visage, pour le cou, pour les mains ou pour le corps (par exemple crème de soin, huile solaire, gel corporel), une composition de maquillage (par exemple gel de maquillage, crème, stick) ou une composition de bronzage artificiel ou de protection de la peau.

La composition selon l'invention peut se présenter sous la forme d'une composition dermatologique ou de soin de la peau et/ou des phanères ou sous forme d'une composition de protection solaire, d'hygiène corporelle, notamment sous forme de déodorant. Elle se présente alors notamment sous forme non colorée. Elle peut alors être utilisée comme base de soin pour la peau, les phanères ou les lèvres (baumes à lèvres, protégeant les lèvres du froid

et/ou du soleil et/ou du vent, crème de soin pour la peau, les ongles ou les cheveux).

Les compositions de l'invention peuvent être obtenues par chauffage des différents constituants à la température de fusion des cires les plus élevées, puis coulage du mélange fondu dans un moule (coupelle ou doigt de gant). Elles peuvent aussi être obtenues par extrusion comme décrit dans la demande EP-A-0 667 146.

**Exemple 1: Rouge à lèvres**

| **Nom chimique** | **Pourcentage massique** |
|---|---|
| Dispersion de polymère acrylate dans le polyisobutène hydrogéné, stabilisé par du Kraton G1701 | 30 |
| Triglycéride d'acide 2-décyl tétradécanoïque | 2.02 |
| Copolymères dimères dilinoleyl diols / dimères dilinoléiques (LUSPLAN DD-DA5®) | 10 |
| Octyldodécanol | 9 |
| conservateur | 0.47 |
| Polycaprolactone de PM 1250 g/mol | 9 |
| Copolymère vinyl pyrrolidone / eicosène | 6 |
| Cire microcristalline | 10 |
| Cire de polyéthylène (PM 500) | 2 |
| Cire de polyméthylène de PF 40°C | 10 |
| Alcool stéarylique | / |
| Pigments | 6.03 |
| Silice enrobée dimethicone | 5 |
| Parfum | 0.48 |
| TOTAL | 100 |

Synthèse de la dispersion de particules de polymère :

On prépare une dispersion de copolymère non réticulé d'acrylate de méthyle et d'acide acrylique dans un rapport 85/15, dans l'heptane, selon la méthode de l'exemple 1 du document EP-A-749 746. Lorsque la polymérisation est terminée, on ajoute du polyisobutène hydrogéné et on distille l'heptane sous vide.
On obtient ainsi une dispersion de particules de poly(acrylate de méthyle/acide acrylique) stabilisées dans le polyisobutène hydrogéné par un copolymère dibloc séquencé polystyrène/copoly(éhylène-propylène) vendu sous le nom de Kraton G1701, ayant un taux de matière sèche de 21% en poids, et une taille moyenne de particule égale à 150 nm.

Mode opératoire du rouge à lèvres :

Toutes les matières premières sont pesées dans un poêlon à double paroi à circulation d'huile, puis mises à chauffer sous agitation (turbine).
Après fonte totale des matières et homogénéisation du mélange, celui-ci est broyé 5 fois de suite à la broyeuse tricylindre. La pâte obtenue est mise à stabiliser pendant 24 heures à 20°C puis conditionnée dans des bouillottes.

**Revendications**

**1.** Composition cosmétique contenant des particules d'au moins un polymère dispersées dans une phase grasse,

- ladite phase grasse étant exempte d'huile volatile ou contenant moins de 50% en poids d'huile volatile par rapport au poids de ladite phase grasse, et

- ladite phase grasse liquide contenant au moins 5% en poids d'au moins une huile peu polaire ou apolaire.

2. Composition selon la revendication précédente, **caractérisée en ce que** la phase grasse contient au moins 10% en poids d'au moins une huile apolaire ou peu polaire.

3. Composition selon la revendication 1 ou 2, **caractérisée en ce que** la phase grasse contient moins de 40%, de préférence moins de 30%, de préférence moins de 20%, de préférence encore moins de 10% en poids d'au moins une huile volatile par rapport au poids total de ladite phase grasse.

4. Composition selon la revendication précédente, **caractérisée en ce qu'**elle est exempte d'huile volatile.

5. Composition selon l'une des revendications précédentes, **caractérisée en ce que** le polymère est tel, que lorsqu'il est dispersé en quantité suffisante dans la phase grasse, la brillance moyenne à 20° d'un dépôt de ladite composition, une fois étalée sur un support, est supérieure ou égale à 30 sur 100.

6. Composition selon la revendication précédente, **caractérisée en ce que** la brillance moyenne mesurée à 20° de la composition, une fois étalée sur un support, est supérieure ou égale à 35, mieux encore, supérieure ou égale à 40, mieux encore, supérieure ou égale à 45, mieux encore, supérieure ou égale à 50, mieux encore, supérieure ou égale à 55, mieux encore, supérieure ou égale à 60.

7. Composition selon l'une des revendications 5 ou 6, **caractérisée en ce que** la brillance moyenne de la composition, une fois étalée sur un support, mesurée à 60° est supérieure ou égale à 50, mieux encore, supérieure ou égale à 60, mieux encore, supérieure ou égale à 65, mieux encore, supérieure ou égale à 70, mieux encore, supérieure ou égale à 75, mieux encore, supérieure ou égale à 80, mieux encore, supérieure ou égale à 85 ou mieux encore, supérieure ou égale à 90 sur 100.

8. Composition selon l'une des revendications 5 à 7, **caractérisée en ce que** la brillance moyenne de la composition, une fois étalée sur un support, mesurée à 20° est supérieure à 35, de préférence 40, 45 ou 50 sur 100, et/ou la brillance moyenne de la composition, une fois étalée sur un support, mesurée à 60° est de préférence supérieure à 65, 70 ou 75 sur 100.

9. Composition selon l'une d es revendications précédentes, **caractérisée en ce que** les particules de polymère sont solides et insolubles dans la phase grasse à une température de 25°C.

10. Composition selon l'une des revendications précédentes, **caractérisée en ce que** le polymère n'est pas une cire.

11. Composition selon l'une des revendications précédentes, **caractérisée en ce que** les particules de polymère ont une taille moyenne comprise entre 5 et 800 nm.

12. Composition selon l'une des revendications précédentes, **caractérisée en ce que** le polymère est filmifiable.

13. Composition selon la revendication précédente, **caractérisée en ce que** le polymère est un polymère hydrocarboné.

14. Composition selon l'une des revendications précédentes, **caractérisée en ce que** les particules de polymère sont insolubles dans les alcools hydrosolubles.

15. Composition selon l'une des revendications précédentes, **caractérisée en ce que** le polymère est choisi parmi les polyuréthanes, polyuréthanes-acryliques, polyurées, polyurée/polyuréthanes, polyester-polyuréthanes, polyéther-polyuréthanes, polyesters, polyesters amides, polyesters à chaîne grasse, alkydes ; polymères ou copolymères acryliques et/ou vinyliques ; copolymères acryliques-silicone ; polyacrylamides ; polymères siliconés, polymères fluorés et leurs mélanges.

16. Composition selon l'une des revendications précédentes, **caractérisée en ce que** le polymère est choisi parmi les copolymères méth(acrylique)s/méthacrylate(s).

17. Composition selon la revendication précédente, **caractérisée en ce que** le polymère est choisi parmi les copolymères acrylique/acrylate tels que le ratio massique des motifs acryliques et des motifs acrylates est compris entre

0,1 et 40%, de préférence entre 2 et 30%, de préférence encore entre 5 et 20%.

**18.** Composition selon l'une des revendications précédentes, **caractérisée en ce que** le polymère représente, en matière sèche, de 5 à 40% du poids total de la composition, de préférence de 5 à 35% et mieux encore de 8 à 30%.

**19.** Composition selon l'une des revendications précédentes, **caractérisée en ce que** la composition comprend un stabilisant choisi parmi les polymères séquencés, les polymères greffés, les polymères statistiques, et leurs mélanges.

**20.** Composition selon la revendication 19, **caractérisée en ce que** le stabilisant est un polymère bloc greffé ou séquencé, comprenant au moins un bloc résultant de la polymérisation de diène et au moins un bloc d'un polymère vinylique.

**21.** Composition selon la revendication précédente, **caractérisée en ce que** le stabilisant est un polymère dibloc.

**22.** Composition selon l'une des revendications précédentes, **caractérisée en ce que** l'huile apolaire ou peu polaire est une huile hydrocarbonée.

**23.** Composition selon la revendication précédente, **caractérisée en ce que** l'huile apolaire ou peu polaire représente de 5% à 80% en poids du poids total de la composition.

**24.** Composition selon la revendication précédente, **caractérisée en ce que** l'huile apolaire ou peu polaire représente de 10% à 60% en poids, de préférence de 1 0% à 40% en poids, de préférence encore de 15 à 30% en poids du poids total de la composition.

**25.** Composition selon l'une des revendications précédente, **caractérisée en ce que** l'huile apolaire ou peu polaire est une huile apolaire hydrocarbonée.

**26.** Composition selon la revendication précédente, **caractérisée en ce que** l'huile apolaire hydrocarbonée est choisies parmi les huiles dont la masse moléculaire est comprise entre 300 et 900 g/mol, de préférence entre 350 et 800 g/mol.

**27.** Composition selon la revendication précédente, **caractérisée en ce que** l'huile apolaire hydrocarbonée non volatile est choisie les hydrocarbures linéaires ou ramifiés tels que les huiles de paraffine, de vaseline et de naphtalène, le polyisobutène hydrogéné, l'isoeicosane, le squalane, les copolymères décène/butène et leurs mélanges.

**28.** Composition selon l'une des revendications précédentes, **caractérisée en ce qu'**elle comprend de 2 à 30 %, de préférence de 5 à 25%, de 5 à 15% d'au moins une huile de masse molaire allant de 650 à 10000 g/mol, et de préférence allant de 750 et 7500 g/mol.

**29.** Composition selon l'une des revendications précédentes, **caractérisée en ce que** qu'elle comprend au moins une matière colorante pulvérulente choisie parmi les pigments, les nacres, les paillettes et leurs mélanges.

**30.** Composition selon la revendication précédente, **caractérisée en ce qu'**elle contient un dispersant choisi parmi le stéarate de l'acide poly(12-hydroxystéarique), l'acide poly (12-hydroxystéarique), le 2-dipolyhydroxystéarate de diglycéryle et leurs mélanges.

**31.** Composition selon l'une des revendications précédentes, **caractérisée en ce qu'**elle se présente sous forme anhydre.

**32.** Composition selon l'une des revendications précédentes, se présentant sous la forme d'un produit de soin et/ou de maquillage de la peau et/ou des lèvres.

**33.** Composition selon l'une des revendications précédentes, se présentant sous forme d'un fond de teint, d'un fard à joues ou à paupières, d'un rouge à lèvres, d'une base ou baume de soin pour les lèvres, d'un produit anti-cernes, d'un eye liner, d'un mascara.

**34.** Utilisation d'une composition selon l'une des revendications 1 à 33 pour obtenir un maquillage brillant, non migrant

et de bonne tenue.